# EUROPEAN PATENT APPLICATION

(11) **EP 2 455 019 A1**
(43) Date of publication of application: **23.05.2012**
(21) Application number: 11189987.8
(22) Date of filing: 21.11.2011
(51) Int. Cl.: A61B 17/34

(54) **Two part thoracic port assembly**

(30) Priority: 22.11.2010 US 415947 P; 08.11.2011 US 291947
(71) Applicant: Tyco Healthcare Group LP, New Haven, CT 06511 (US)
(72) Inventor: Haig, Fiona Middlemiss, Boston, Massachusetts 02116 (US); Collier, John Nicholas, Cambridge, CB25 9JG (GB)
(74) Representative: Soames, Candida Jane

(57) **Abstract**

A surgical port (10) for use during a thoracic surgery includes a first port element (12) and a second port element (14). The first port element includes a first sidewall (16) and a first flange (18) that is positioned adjacent a proximal portion of the first sidewall. The first flange extends in a laterally outward direction. The second port element includes a second sidewall (20) and a second flange (22) that is positioned adjacent a distal portion of the second sidewall. The second flange extends in a laterally outward direction. The first port element is configured to securely couple to the second port element and each of the first and second sidewalls defines a passageway to allow a surgical instrument to pass therethrough.

## Description

### BACKGROUND

This application claims priority from provisional application serial no. 61/415,947, filed November 22, 2010, the entire contents of which are incorporated herein by reference.

### Technical Field

The present disclosure relates to a thoracic port assembly for use during a thoracic surgical procedure. More particularly, the present disclosure relates to a thoracic port assembly which includes first and second port elements that are coupled to each other for use during the thoracic surgical procedure.

### Description of Related Art

In an effort to reduce trauma and recovery time, many surgical procedures are performed through small openings in the skin, such as an incision or a natural body orifice. For example, these procedures include laparoscopic procedures, which are generally performed within the confines of a patient's abdomen, and thoracic procedures, which are generally performed within a patient's chest cavity.

Specific surgical instruments have been developed for use during such minimally invasive surgical procedures. These surgical instruments typically include an elongated shaft with operative structure positioned at a distal end thereof, such as graspers, clip appliers, specimen retrieval bags, etc.

During minimally invasive procedures, the clinician creates an opening in the patient's body wall, oftentimes by using an obturator or trocar, and thereafter positions an access assembly within the opening. The access assembly includes a passageway extending therethrough to receive one or more of the above-mentioned surgical instruments for positioning within the internal work site, e.g. the body cavity.

During minimally invasive thoracic procedures, an access assembly is generally inserted into a space located between the patient's adjacent ribs that is known as the intercostal space, and then surgical instruments are inserted into the internal work site through the passageway in the access assembly.

In the interests of facilitating visualization, the introduction of certain surgical instruments, and/or the removal of tissue specimens during minimally invasive thoracic procedures, it may be desirable to spread/retract the tissue adjacent the ribs defining the intercostal space. Additionally, during these procedures, firm, reliable placement of the access assembly is desirable to allow the access assembly to withstand forces that are applied during manipulation of the instrument(s) inserted therethrough. However, reducing patient trauma during the procedure, discomfort during recovery, and the overall recovery time remain issues of importance. Thus, there exists a need for a thoracic access port which minimizes post operative patient pain while enabling atraumatic retraction of tissue and which does not restrict access to the body cavity as well as facilitates removal of tissue specimens from the body cavity.

### SUMMARY

The present disclosure relates to a surgical port for use during a thoracic surgical procedure. The surgical port includes a first proximal port element and a second distal port element. The first port element includes a first sidewall and a first flange that is positioned adjacent a proximal portion of the first sidewall. The first flange extends in a laterally outward direction. The second port element includes a second sidewall and a second flange that is positioned adjacent a distal portion of the second sidewall. The second flange extends in a laterally outward direction. The first port element is configured to securely couple to the second port element and each of the first and second sidewalls defines a passageway to allow a surgical instrument to pass therethrough.

Each of the first and second sidewalls may include an interior portion and an exterior portion. In some embodiments, the first port element is configured to securely and removably fit over the second port element such that the interior portion of the first sidewall is configured to engage the exterior portion of the second sidewall in a securing manner. In other embodiments, the first port element is configured to securely and removably fit within the second port element such that the interior portion of the second sidewall is configured to engage the exterior portion of the first sidewall in a securing manner.

The first flange can in some embodiments be positioned along a top periphery, in other embodiments it can be positioned along a bottom periphery.

In some embodiments, the first and second port elements are selectively couplable so the distance between the first and second port elements can vary when coupled.

In some embodiments, the first and second port elements are frictionally secured.

### BRIEF DESCRIPTION OF THE DRAWINGS

Various embodiments of the port assembly are described herein with reference to the drawings wherein:

FIG. 1 is a perspective view of a thoracic port assembly, with parts separated, in accordance with an embodiment of the present disclosure;

Figure 1A is a side, cross-sectional view of the thoracic port assembly of FIG. 1 positioned between two ribs of a patient during a surgical procedure; and

FIG. 2 is a side, cross-sectional view of the thoracic port assembly of an alternate embodiment positioned between two ribs of a patient during a surgical procedure.

### DETAILED DESCRIPTION

Embodiments of the presently disclosed port assembly are described in detail with reference to the drawings wherein like reference numerals identify similar or identical elements. As used herein, the term "distal" refers to that portion of the device which is further from a user, while the term "proximal" refers to that portion of the device which is closer to a user.

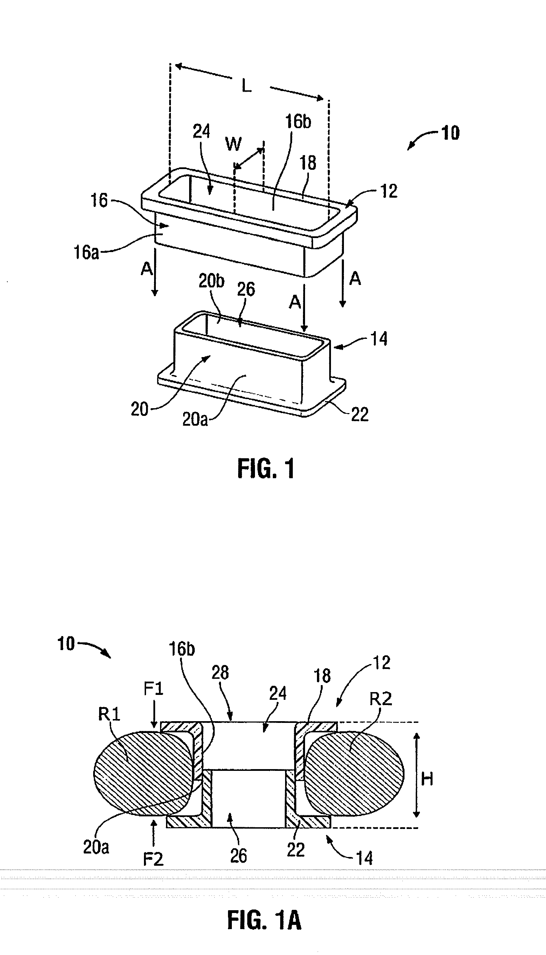

Referring initially to FIG. 1, there is disclosed an embodiment of a thoracic port assembly 10 for use during a thoracic surgical procedure. It should be appreciated that the access port assembly can also be used for other procedures and/or provide access to other regions of the body. Port assembly 10 generally includes a top (first) or proximal port element 12 and a bottom (second) or distal port element 14. Port assembly 10 is configured and dimensioned to have a substantially box-like configuration, for example, including a configuration that is substantially rectangular, square, oval, circular, etc.

Top or external port element 12 includes a sidewall 16 and a securing flange 18. Sidewall 16 defines an orifice or passageway 24 to allow a surgical instrument (not shown) to pass therethrough. Sidewall 16 is configured to retain the surgical instrument within orifice 24 during a surgical procedure. Securing flange 18 is positioned around a proximal portion and preferably around a top periphery of sidewall 16 and extends in a laterally, outwardly direction, thereby having a transverse dimension greater than a transverse dimension of the sidewall 16. Securing flange 18 is configured to secure top port element 12 at a desired position during a surgical procedure, as will be described in further detail below.

Similar to top port element 12, bottom port element 14 includes a sidewall 20 and a securing flange 22. Sidewall 20 defines an orifice or passageway 26 to allow a surgical instrument (not shown) to pass therethrough. Sidewall 20 is configured to retain the surgical instrument within orifice 26 during a surgical procedure. Securing flange 22 is positioned around a distal portion and preferably around a bottom periphery of sidewall 20 and extends in a laterally, outwardly direction, thereby having a transverse dimension greater than a transverse dimension of sidewall 26. Securing flange 22 is configured to secure bottom port element 14 at a desired position during a surgical procedure, as will be described in further detail below.

Both sidewalls 16 and 20 include an exterior portion 16a, 20a, respectively, and an interior portion 16b, 20b, respectively. Interior portion 16b, 20b of sidewall 16, 20 may have a length, as indicated by arrows "L," greater than a width, as indicated by arrows "W." In addition, port assembly 10 may have a variable height, as indicated by arrows "H," as will be described in detail further below. The sidewalls 16, 20 or any of the walls thereof can be substantially planar or can have an arcuate surface.

Top port element 12 is configured to securely and removably fit over bottom or internal port element 14, as shown by directional arrow "A." More specifically, interior portion 16b of sidewall 16 is configured to engage exterior portion 20a of sidewall 20 in a securing manner. (The width of interior portion 16b is greater than the width of the exterior portion 20a). It is envisioned that any suitable type of securing technique may be utilized to securely engage top port element 12 to bottom port element 14. For example, top port element 12 may be secured to bottom port element 14 by any suitable fastening technique, for example, but not limited to friction-fit, snap-fit, and fluid-fit (e.g., a viscous fluid coating).

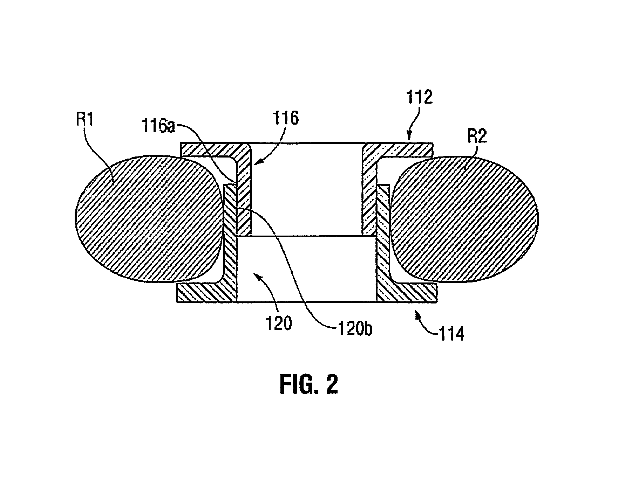

In an alternative embodiment, the top port element may be configured to securely and removably fit within the bottom port element in the same direction as directional arrows "A." This is illustrated in the alternate embodiment of Figure 2. In this embodiment, exterior portion 116a of sidewall 116 of the top port element of 112 is configured to engage interior portion 120b of sidewall 120 of the bottom port element 114 in a securing manner (the width of exterior portion 116a is less than the width of interior portion 120b). Otherwise, the port elements 112 and 114 function in the same way and are inserted in the same way as the port elements of port assembly 10 of Figure 1. Any suitable type of securing technique can be utilized to secure top and bottom port elements 112 and 114 such as a friction fit, snap-fit, fluid-fit, etc.

Referring now to FIG. 1A, port assembly 10 is shown positioned between a first rib "R1" and a second rib "R2" of a patient. As described above, in preferred embodiments, port assembly 10 is configured to have an adjustable height "H." That is, top port element 12 and bottom port element 14 may be adjusted with respect to each other by a variable distance to thereby adjust the height "H." In this manner, thoracic port 10 can be used in different situations where the height "H" of thoracic port 10 is required to be adjusted. For example, if the ribs "R1" and "R2" and/or adjacent tissue of a patient have a larger dimension, then top port element 12 and bottom port element 14 will be adjusted to have a larger height "H," so that securing flanges 18 and 22 may be securely retained in the tissue. Thus, the distance between top port element 12 and bottom port element 14 will be increased. In another example, if the ribs "R1" and "R2" and/or adjacent tissue of a patient are of smaller dimension, then top port element 12 and bottom port element 14 will be adjusted to have a smaller height "H," as securing flanges 18 and 22 can be moved closer together. That is, the distance between top port element 12 and bottom port element 14 will be decreased.

The port assembly of FIGS. 1 and 2 may be fabricated from any biocompatible material with a strength suitable for the purposes described herein. The port assembly may be rigid enough to resist excessive bending under the conditions normally encountered in connection with the use described herein. In addition, port assembly may be rigid enough for providing an imparting force sufficient to maintain the ribs "R1" and "R2" in a desired position to accommodate insertion of surgical instruments within port assembly 10 and in some embodiments rigid enough to impact force sufficient to spread (retract) tissue adjacent the ribs and/or the ribs during insertion of the port in the intercostal space.

Port assembly 10 (and the port assembly of FIG. 2), due to its adjustable height, may be configured to provide a suitable retaining force that is exerted against the ribs "R1" and "R2" during a surgical procedure to maintain the port in place. That is, flange 18 of top port element 12 may provide a downward force "F1," while flange 22 may provide an upward force "F2,", the downward force "F1" and upward force "F2" facilitating positioning and retaining of port assembly 10 in a desired positioned selected by a user, e.g., a surgeon.

When port assembly 10 is in an assembled configuration, i.e., when top port element 12 is coupled to bottom port element 14, orifices (passageways) 24 and 26 provide a passageway 28 between top port element 12 and bottom port element 14 such that a user, e.g., a surgeon, may insert a surgical instrument (not shown) therethrough to access the body cavity, e.g. thoracic cavity, to perform a surgical procedure.

With continued reference to FIG. 1A, insertion and removal of port assembly 10 will now described. The port assembly of FIG. 2 would be inserted in the same manner. Initially, an introducer and/or trocar assembly (not shown) is used to penetrate the body cavity of the patient to access the intercostal space, which is the area between ribs "R1" and "R2" of the patient. After access is provided to the intercostal space, bottom port element 14 is introduced and positioned within the intercostal space, and ribs "R1" and "R2." Port element 14 is preferably inserted lengthwise through the incision and then rotated to the transverse position of Figure 1A. The port element 14 (and 114) can also be flexible to facilitate insertion. A ribbon or other structure can be provided (not shown) attached to bottom port element 14 (and 114), e.g. to exterior portion 20a or flange 22, and extending outside the body cavity to be grasped by the user to hold the bottom port element 14 as the top port element is attached. After insertion of the bottom element 14 into the body cavity, top port element 12 is introduced and the sidewall 16 advanced within the intercostal space between ribs "R1" and "R2" so top port element 12 engages bottom port element 14 to provide a secure connection. As discussed above, flange 18 of top port element 12, dimensioned greater than the dimension of the intercostal space, may provide a downward force "F1," while flange 22, also dimensioned larger than the dimension of the intercostal space, may provide an upward force "F2." The downward force "F1" and upward force "F2" facilitate positioning and retaining of port assembly 10 in a desired position. Flanges 18 and 22, respectively, also prevent the top port element 12 from unintentionally sliding into the cavity and prevent bottom port element 14 from sliding out of the body cavity. In this configuration, port assembly 10 provides a surgeon access to a surgical site beneath the intercostal space to insert instrumentation through the passageway extending therethrough. Note the extent of insertion of the top element 12 into or over the bottom element 14 is dependent on the size/height of the ribs and surrounding tissue. The port assembly of FIG. 2 operates in the same fashion (and the foregoing discussion is applicable to the port of FIG. 2), except that for securing the top and bottom port elements, top port element 116 is moved within bottom port element 114.

After a surgical procedure has been completed, port assembly 10 is removed from within the intercostal space. More specifically, top port element 12 (or 112) is separated from bottom port element 14 (or 114) and removed from the intercostal space and then bottom port element 14 is removed from the cavity by for example pulling the ribbon or by an alternate surgical tool, first angling/tilting the port element 14.

It should be noted that any suitable type of instrument(s) (e.g., a Kelly clamp) may be used to facilitate positioning of top and bottom port elements 12 (112) and 14 (114) during insertion and removal of port assembly 10 within the intercostal space.

It will be understood that various modifications may be made to the embodiments disclose herein. Therefore, the above description should not be construed as limiting, but merely as exemplifications of embodiments. Those skilled in the art will envision other modification within the scope and spirit of the claims appended hereto.
The invention may be described by reference to the following numbered paragraphs:-
1. A surgical port for use during a thoracic surgical procedure, the surgical port comprising:
   a first proximal port element having a first sidewall and a first flange positioned adjacent a proximal portion of the first sidewall, the first flange extending in a laterally outward direction; and
   a second distal port element having a second sidewall and a second flange positioned adjacent a distal portion of the second sidewall, the second flange extending in a laterally outward direction, the first port element being configured to securely couple to the second port element and each of the first and second sidewalls defining a passageway to allow a surgical instrument to pass therethrough.
2. The surgical port according to paragraph 1, wherein the port is configured and dimensioned to have a substantially box-like configuration.
3. The surgical port according to paragraph 2, wherein the substantially box-like configuration of the port assembly is selected from the group consisting of rectangular, square, and circular.
4. The surgical port according to paragraph 1, wherein the first flange is positioned along a top periphery of the first port element.
5. The surgical port according to paragraph 1, wherein the second flange is positioned along a bottom periphery of the second port element.
6. The surgical port according to paragraph 5, wherein the first flange is positioned along a top periphery of the first port element.
7. The surgical port according to paragraph 1, wherein the first port element is configured to securely and removably fit over the second port element such that an interior portion of the first sidewall is configured to engage an exterior portion of the second sidewall in a securing manner.
8. The surgical port according to paragraph 1, wherein the first port element is configured to securely and removably fit within the second port element such that an interior portion of the second sidewall is configured to engage an exterior portion of the first sidewall in a securing manner.
9. The surgical port according to paragraph 1, wherein the first and second port elements are selectively couplable so the distance between the first and second port elements can vary when coupled.
10. The surgical port according to paragraph 6, wherein the first port element is configured to securely and removably fit over the second port element such that an interior portion of the first sidewall is configured to engage an exterior portion of the second sidewall in a securing manner.
11. The surgical port according to paragraph 6, wherein the first port element is configured to securely and removably fit within the second port element such that an interior portion of the second sidewall is configured to engage an exterior portion of the first sidewall in a securing manner.
12. The surgical port according to paragraph 1, wherein a first width of the first sidewall is less than a second width of the second sidewall.
13. The surgical port according to paragraph 1, wherein a first width of the first sidewall is greater than a second width of the second sidewall.
14. The surgical port according to paragraph 7, wherein the first and second port elements are frictionally secured.
15. The surgical port according to paragraph 8, wherein the first and second port elements are frictionally secured.

## Claims

1. A surgical port for use during a thoracic surgical procedure, the surgical port comprising:
a first proximal port element having a first sidewall and a first flange positioned adjacent a proximal portion of the first sidewall, the first flange extending in a laterally outward direction; and
a second distal port element having a second sidewall and a second flange positioned adjacent a distal portion of the second sidewall, the second flange extending in a laterally outward direction, the first port element being configured to securely couple to the second port element and each of the first and second sidewalls defining a passageway to allow a surgical instrument to pass therethrough.

2. The surgical port according to Claim 1, wherein the port is configured and dimensioned to have a substantially box-like configuration.

3. The surgical port according to Claim 2, wherein the substantially box-like configuration of the port assembly is selected from the group consisting of rectangular, square, and circular.

4. The surgical port according to any preceding claim, wherein the first flange is positioned along a top periphery of the first port element.

5. The surgical port according to any preceding claim, wherein the second flange is positioned along a bottom periphery of the second port element.

6. The surgical port according to any preceding claim, wherein the first port element is configured to securely and removably fit over the second port element such that an interior portion of the first sidewall is configured to engage an exterior portion of the second sidewall in a securing manner.

7. The surgical port according to any preceding claim, wherein the first port element is configured to securely and removably fit within the second port element such that an interior portion of the second sidewall is configured to engage an exterior portion of the first sidewall in a securing manner.

8. The surgical port according to Claim 1, wherein the first and second port elements are selectively couplable so the distance between the first and second port elements can vary when coupled.

9. The surgical port according to any preceding claim, wherein a first width of the first sidewall is less than a second width of the second sidewall.

10. The surgical port according to any preceding claim, wherein the first and second port elements are frictionally secured.

11. The surgical port according to any preceding claim, wherein a first width of the first sidewall is greater than a second width of the second sidewall.
